# DEMANDE DE BREVET EUROPEEN

(11) **EP 4 474 004 A1**
(43) Date de publication de la demande: **11.12.2024**
(21) Numéro de dépôt: 24180045.7
(22) Date de dépôt: 04.06.2024
(51) Int. Cl.: A61N 5/06

(54) **DISPOSITIF DE TRAITEMENT PAR PHOTOTHÉRAPIE**

(30) Priorité: 07.06.2023 FR 2305751
(71) Demandeur: Lucibel SA, 76360 Barentin (FR)
(72) Inventeur: GROS-DAILLON, Thibault, 76590 CRIQUETOT-SUR-LONGUEVILLE (FR); MOA, Yacine, 76510 Saint Nicolas d'Aliermont (FR)
(74) Mandataire: Martin, Marie-Aude

(57) **Abrégé**

Le casque (10) de traitement du cuir chevelu d'un utilisateur par photothérapie, le casque (10) comprenant des parois de coque extérieure (22) et intérieure (24) s'étendant chacune d'une portion de sommet proximale à un bord distal (220, 240) et définissant une fois assemblées, une bordure périphérique du casque (10) qui délimite une ouverture de passage de la tête de l'utilisateur et une source de lumière disposée entre les deux parois (22, 24) émettant vers l'intérieur du casque (10). Selon l'invention, le casque (10) comprend un bandeau circonférentiel (40) d'ajustement du casque (10) à un tour de tête de l'utilisateur, accroché à la bordure périphérique, ledit bandeau (40) comprenant un corps pourvu d'une portion de déformation (46) s'étendant radialement vers l'intérieur du casque (10) et étant configurée pour se déformer entre une position de repos déployée radialement vers l'intérieur du casque (10) et une position tassée entre le casque et la tête de l'utilisateur en configuration d'utilisation.

## Description

La présente invention concerne un dispositif de traitement par photothérapie pour une région externe du corps d'un être vivant. Plus particulièrement mais non exclusivement, elle s'applique au traitement par photothérapie de la surface de tête d'un utilisateur, par exemple au traitement du cuir chevelu en tant que traitement de la calvitie.

De façon connue en soi, le traitement consiste en l'application d'un rayonnement lumineux présentant un spectre d'émission prédéfini directement sur le cuir chevelu. Ce traitement par photothérapie peut être appliqué également en combinaison avec un produit photo-sensibilisant appliqué sur la région à traiter quelques heures avant son exposition au rayonnement lumineux pour augmenter l'efficacité du traitement. On parle alors dans ce cas de photothérapie dynamique (connue également sous l'acronyme anglais PDT « Photo Dynamic Therapy »).

Le rayonnement est de préférence sensiblement monochromatique ou présente un spectre d'émission compris dans une bande relativement étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau.

Il a été ainsi observé au cours de nombreuses études scientifiques, qu'un traitement par photothérapie dans une plage de longueurs d'onde prédéfinie, notamment dans la lumière rouge ou bleue, permet d'agir de façon bénéfique sur de nombreux problèmes cutanés, tels que les cicatrices, les vergetures, l'acné, les rides et ridules.

Plus particulièrement, lors de l'application du rayonnement sur la peau, notamment dans la lumière rouge et plus particulièrement autour de la longueur d'onde 630 nm, les cellules de la région irradiées vont réagir selon un principe similaire à celui de la photosynthèse des végétaux. La lumière va stimuler les cellules, et plus spécifiquement certaines structures cellulaires de la cellule, telles que les mitochondries.

De façon connue en soi, les mitochondries jouent un rôle particulièrement important dans le processus de respiration cellulaire ainsi que dans le processus de transformation des nutriments en un vecteur énergétique désigné couramment par ATP (acronyme pour Adénosine Tri Phosphate). Dès lors, la stimulation des mitochondries par la lumière rouge contribue à une régénération des cellules grâce à l'amélioration du métabolisme cellulaire. Une telle régénération cellulaire va se manifester par exemple pour les cellules de la peau du visage par une augmentation de la production de collagène et d'élastine et par conséquent une diminution observable des rides et ridules.

Plus particulièrement, il a été observé que l'exposition du cuir chevelu à la lumière rouge, par exemple à la longueur d'onde de 630 nm, permet une stimulation de la repousse capillaire, une amélioration de la qualité et de la densité des cheveux ainsi qu'une stabilisation de la perte de cheveux.

On connaît déjà dans l'état de la technique, un équipement de traitement par photothérapie du cuir chevelu d'un utilisateur, comprenant un casque délimitant une extrémité ouverte par laquelle, en position d'utilisation, la tête de l'utilisateur est au moins partiellement engagée.

Afin de maintenir le casque sur la tête pendant le traitement de photothérapie et ce indépendamment de la taille du tour de tête de l'utilisateur, plusieurs solutions ont été proposées dans l'art antérieur.

Par exemple, une des solutions proposées est de maintenir le casque grâce à des unités d'oreille qui viennent enserrer la tête de chaque côté au niveau des oreilles, comme dans la demande de brevet EP2477697.

Cette solution présente l'inconvénient de perturber l'environnement sonore de l'utilisateur pendant son traitement de photothérapie ce qui présente un inconvénient lorsque ce dernier souhaite participer par exemple à une conversation avec un interlocuteur.

Une autre solution est de prévoir une sangle jugulaire qui vient retenir le casque au niveau du menton de l'utilisateur.

Toutefois, en plus d'être inesthétique, cette solution est peu confortable pour l'utilisateur dont le menton est comprimé pendant le traitement.

### Description de l'invention

La présente invention a notamment pour but de proposer un dispositif de photothérapie qui remédie à ses inconvénients.

A cet effet l'invention a pour objet un casque de traitement du cuir chevelu d'un utilisateur par photothérapie, le casque comprenant des parois de coque extérieure et intérieure s'étendant chacune d'une portion de sommet proximale à un bord distal et définissant une fois assemblées, une bordure périphérique du casque qui délimite une ouverture de passage de la tête de l'utilisateur et une source de lumière disposée entre les deux parois émettant vers l'intérieur du casque, caractérisé en ce qu'il comprend un bandeau circonférentiel d'ajustement du casque à un tour de tête de l'utilisateur, accroché à la bordure périphérique, ledit bandeau comprenant un corps pourvu d'une portion de déformation s'étendant radialement vers l'intérieur du casque et étant configurée pour se déformer entre une position de repos déployée radialement vers l'intérieur du casque et une position tassée entre le casque et la tête de l'utilisateur en configuration d'utilisation.

Un casque selon l'invention peut comprendre l'une ou plusieurs des caractéristiques suivantes.

Dans un mode de réalisation de l'invention, la portion de déformation est creuse.

Dans un mode de réalisation de l'invention, le bandeau est monté sur le casque de telle sorte que le bandeau ne peut être démonté avant démontage des deux parois de coque entre elles.

Dans un mode de réalisation de l'invention, la portion de déformation se prolonge vers l'intérieur du casque par un bourrelet de rattachement qui est pincé entre des reliefs des bords distaux des parois de coque.

Dans un mode de réalisation de l'invention, la paroi intérieure comprend sur sa face arrière une nervure en saillie circonférentielle qui forme un épaulement d'appui au niveau du bord distal de telle sorte que le bourrelet est pris en étau à l'intérieur de cet épaulement par la paroi de coque intérieure contre un relief de la paroi de coque extérieure.

Dans un mode de réalisation de l'invention, le bord distal de la paroi de coque extérieure est replié en extrémité en saillie vers l'intérieur pour former un rebord s'étendant radialement vers l'intérieur pour former un épaulement d'appui de telle sorte que le bourrelet est pris en étau à l'intérieur de cet épaulement par la paroi de coque extérieure contre un relief de la paroi de coque intérieure.

Dans un mode de réalisation de l'invention, la portion de déformation se prolonge vers l'extérieur du casque par un talon d'ancrage extérieur qui est retenu par un relief ménagé sur une face arrière de la paroi de coque extérieure.

Dans un mode de réalisation de l'invention, le talon d'ancrage est formé par une bordure d'extrémité libre recourbée en U qui vient s'encastrer radialement dans une rainure circonférentielle ménagée sur une face extérieure de la paroi de coque extérieure.

Dans un mode de réalisation de l'invention, le bandeau d'ajustement est configuré pour être prépositionné en étant retenu de façon libérable sur la paroi de coque extérieure dans une position de destination finale, avant assemblage de la paroi de coque intérieure à la paroi de coque extérieure pour bloquer le bandeau dans ladite position.

Dans un mode de réalisation de l'invention, le bandeau est réalisé dans un matériau déformable et résilient de dureté shore A comprise entre 25 et 95 shore.

Dans un mode de réalisation de l'invention, le casque comprend une plaque d'interface de dissipation thermique réalisée dans un matériau opaque à la lumière qui s'étend entre les deux parois de coque.

Dans un mode de réalisation de l'invention, le bandeau, la paroi de coque extérieure étant réalisées dans des matériaux translucides, la paroi de coque extérieure présente sur son bord distal un motif en surépaisseur locale qui empêche localement la lumière de passer de façon à produire un motif lumineux.

Dans un mode de réalisation de l'invention, les deux parois de coque sont assemblées l'une avec l'autre par des moyens de liaison permanents ou libérales.

L'invention a encore pour objet un ensemble comprenant un casque selon l'invention et un groupe de bandeaux avec une pluralité de circonférences intérieures différentes définissant autant de catégories dimensionnelles différentes, le bandeau du casque étant choisi dans ledit groupe et assemblé sur le casque pour définir la catégorie dimensionnelle du casque.

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig. 1] La [Fig.1] est une vue en perspective d'un casque selon l'invention pour le traitement du cuir chevelu d'un utilisateur ;
[Fig.2] La [Fig.2] est une vue de dessous du casque de la [Fig.2] ;
[Fig.3] La [Fig.3] est une vue en coupe transversale selon la ligne III-III de la [Fig.1]
[Fig.4] La [Fig.4] est vue en perspective d'une interface de dissipation thermique sur laquelle est montée une source de lumière ;
[Fig.5] La [Fig.5] est une vue éclatée en perspective du casque ;
[Fig.6] La [Fig.6] est une vue en perspective d'un bandeau d'ajustement du casque de la [Fig.5] ;
[Fig.7] La [Fig.7] est une vue en coupe transversale du bandeau de la [Fig.6].

### Description détaillée de l'invention

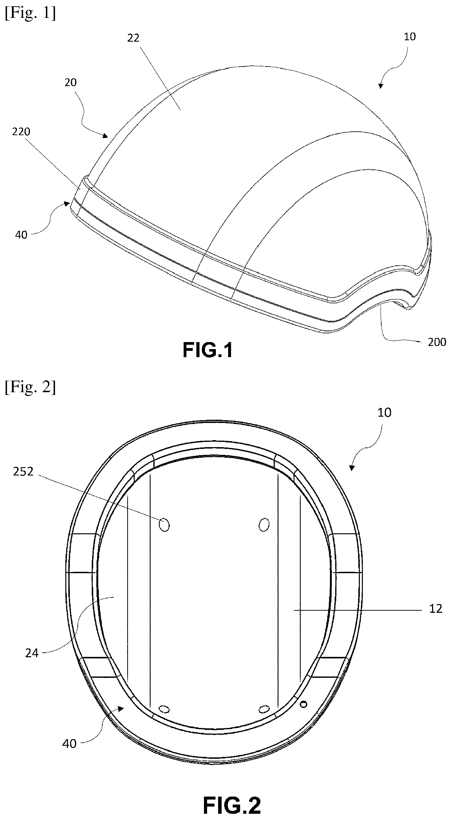

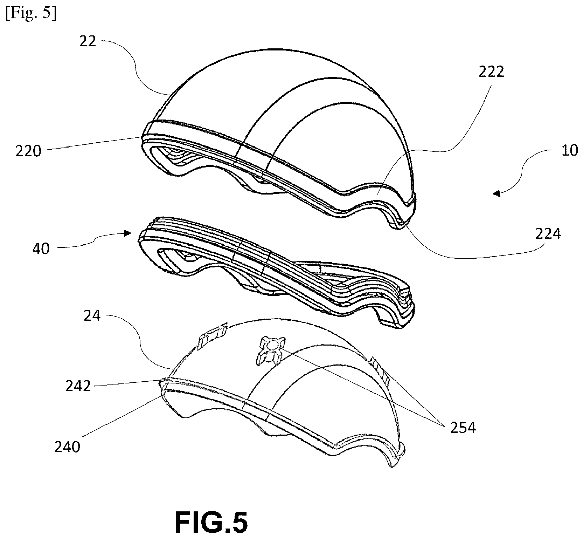

On a représenté sur les figures 1 à 7 un casque de traitement du cuir chevelu selon l'invention. Le casque de traitement est désigné par la référence générale 10.

Comme illustré sur les figures 1 à 3, le casque de traitement 10 comprend un corps 20 qui délimite une cavité 12 à l'intérieur de laquelle un utilisateur peut insérer sa tête.

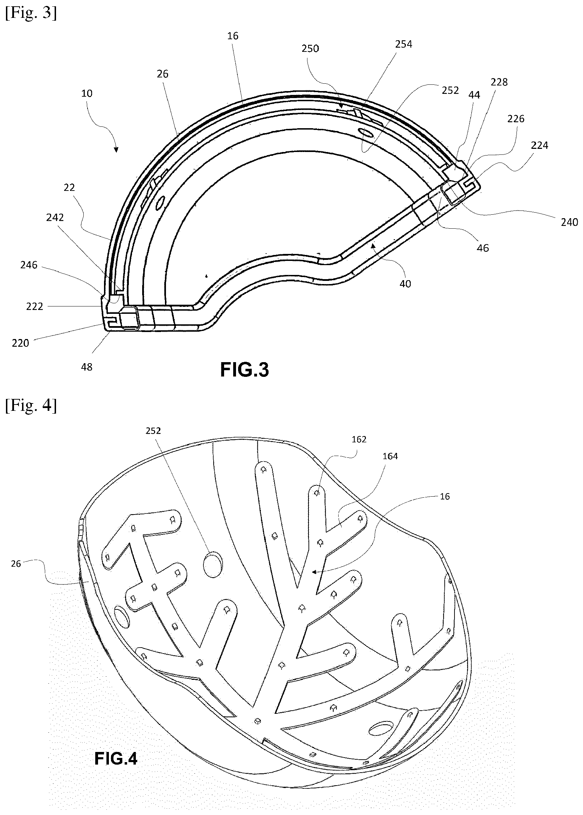

Le corps 20 incorpore encore une source de lumière 16 qui émet vers l'intérieur de la cavité 12 en direction de la surface à traiter. La source de lumière 16 comprend dans cet exemple, tel qu'illustré sur la [Fig.4], une pluralité de diodes électroluminescentes 162. Dans l'exemple décrit, les diodes électroluminescentes 162 émettent une lumière rouge de préférence avec une longueur d'onde autour de 630 nanomètres, dans une plage de 620 à 640 nanomètres. En variante, d'autres longueurs d'onde de lumière peuvent être sélectionnées en fonction de la nature du traitement de photothérapie souhaité. Par exemple, les diodes électroluminescentes 162 sont portées par un support d'interconnexion électrique 164.

Comme cela est illustré sur les figures, le corps 20 du casque 10 comprend des parois de coque extérieure 22 et intérieure 24 s'étendant chacune d'une portion de sommet proximale jusqu'à un bord distal 220, 240 de la paroi de coque 22, 24. Les deux parois de coque 22, 24, définissent une fois assemblées, une bordure périphérique 200 du casque 10 qui délimite l'ouverture de passage de la tête d'un utilisateur.

La paroi de coque intérieure 24 est réalisée de préférence dans une matière laissant passer le flux lumineux émis par la source de lumière 16. De préférence, la matière est translucide pour permettre une diffusion homogène de la lumière ainsi qu'une protection naturelle de l'utilisateur contre l'éblouissement. Par translucide, on entend un matériau qui laisse passer la lumière mais qui n'est pas nécessairement transparent. Le choix d'un matériau translucide plutôt qu'un matériau transparent permet également au niveau esthétique de rendre moins visibles les diodes de la source lumineuse. Bien entendu, en variante, le matériau peut être choisi transparent aux rayons lumineux émis par les sources lumineuses. La paroi de coque intérieure 24 peut être également désignée par vitre intérieure du fait de ses propriétés de transmission lumineuse.

Par exemple, de préférence, la paroi intérieure 24 est réalisée dans une matière plastique thermoformable, par exemple un mélange de polystyrène et de polyéthylène (acronyme PS-PE).

Dans le mode de réalisation préféré de l'invention, la source de lumière 16 est disposée entre les deux parois de coque 22, 24 et émet vers l'intérieur du casque 10.

Selon l'invention, le casque 10 comprend encore un bandeau circonférentiel 40 d'ajustement du casque 10 à un tour de tête de l'utilisateur, accroché à la bordure périphérique 200. Ce bandeau 40 permet d'ajuster la cavité 12 du casque 10 au tour de tête de l'utilisateur, ce tour de tête présentant une grande variabilité au sein de la population générale.

En particulier, ce bandeau 40 comprend un corps pourvu d'une portion de déformation 46 s'étendant radialement vers l'intérieur du casque 10 qui est configurée pour se déformer entre une position de repos déployée radialement vers l'intérieur du casque 10 et une position tassée entre le casque 10 et la tête de l'utilisateur en configuration d'utilisation. De préférence, cette portion de déformation 46 est creuse ce qui permet, par exemple, de guider la déformation selon une ligne de déformation préférentielle.

Dans l'exemple décrit, le bandeau 40 est maintenu accroché au casque après assemblage des deux parois de coque 22, 24 entre elles. Le bandeau 40 comprend dans l'exemple décrit deux portions de rattachement, une première portion de rattachement 48 à l'extérieur du casque 10 et une deuxième portion de rattachement 44 à l'intérieur du casque 10.

A cet effet, la portion de déformation 46 se prolonge vers l'intérieur du casque 10 par un bourrelet périphérique de rattachement 44 qui forme la deuxième partie de rattachement. Ce bourrelet 44 est de préférence pincé entre des reliefs des bords distaux 220, 240 des parois de coque 22 et 24.

Dans un mode de réalisation préféré de l'invention, la portion de déformation 46 se prolonge vers l'extérieur du casque 10 par un talon périphérique 48 d'ancrage extérieur formant la première partie de rattachement du bandeau 40. Ce talon 48 est de préférence retenu par encastrement dans un relief ménagé sur une face arrière de la paroi de coque extérieure 22, par exemple dans le bord distal 220.

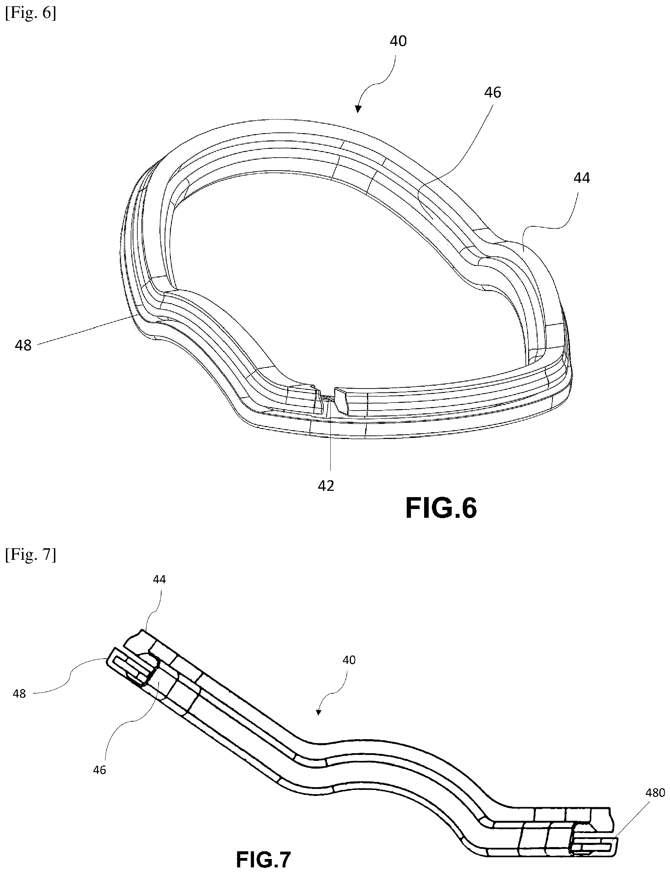

Par exemple, la première portion de rattachement 48 est logée dans une rainure circonférentielle 224 ménagée sur la face externe de la paroi de casque extérieure 22. Dans le mode de réalisation préféré de l'invention, le bandeau 40 comprend une bordure libre 480 recourbée en U ([Fig.7]) qui vient s'encastrer radialement dans la rainure circonférentielle 224 ménagée sur la face extérieure de la paroi de coque 22.

De préférence, la paroi de coque intérieure 24 comprend sur sa face arrière une nervure en saillie circonférentielle 242 qui forme un épaulement d'appui 244 au niveau du bord distal 240 de telle sorte que le bourrelet 44 du bandeau 40 est pris en étau à l'intérieur de cet épaulement 244 par la paroi de coque intérieure 24 contre un relief de la paroi de coque extérieure 22.

Dans l'exemple décrit, le bord distal 220 de la paroi extérieure 22 est replié en extrémité en saillie vers l'intérieur pour former un rebord 226 s'étendant radialement vers l'intérieur en formant un épaulement d'appui 228 de telle sorte que le bourrelet 44 est pris en étau à l'intérieur de cet épaulement 228 par la paroi de coque extérieure 22 contre un relief de la paroi de coque intérieure 24.

En variante, la portion de déformation 46 peut avoir une section transversale pleine ou creuse et de forme variée, par exemple de forme choisie dans la liste suivante : polygonale, carrée, rectangulaire, triangulaire, circulaire, non circulaire, oblongue, ovale, elliptique, crénelée, en forme d'étoile, etc.

Par exemple, le bandeau 40 est réalisé dans un matériau déformable et résilient de dureté shore A comprise entre 25 et 95 shore, de préférence 50 shore.

De préférence, le bandeau 40 est réalisé dans une matière comprenant un élastomère de polyuréthane ou de silicone. En outre, de préférence, le bandeau 40 présente un passage 42 pour un câble d'alimentation électrique de la source 16. Par exemple, la portion de rattachement 44 est localement interrompue pour former le passage 42.

Le casque 10 comprend encore dans l'exemple illustré, sur sa bordure périphérique 200 deux échancrures correspondant aux zones de contournement des oreilles. Comme illustré, le bandeau 40 a une forme générale d'arceau présentant un profil circonférentiel curviligne avec de préférence deux échancrures de contournement de l'oreille. Ceci permet de dégager les oreilles de l'utilisateur en configuration d'utilisation.

De préférence, la coque extérieure 22 est pourvue sur sa face interne d'une extrémité s'évase vers l'extérieur pour former une collerette 222 avant de se recourber vers l'intérieur par son rebord 226,

En outre, le casque 10 comprend encore une couche de dissipation thermique 26 intercalée entre les deux parois de coque 22, 24. De préférence, la couche 26 est conformée en une plaque configurée pour s'étendre dans le corps du casque 10. Comme cela est illustré en figure, la plaque 26 forme un support pour la source de lumière. Dans l'exemple illustré, la source de lumière 16 comprend un support flexible 164 et une pluralité de diodes électroluminescentes 162. Le support flexible 164 a par exemple une configuration étoilée afin de se conformer à la concavité des parois de coque 22, 24. De préférence, le support 164 est rapporté sur une face interne de la plaque de dissipation thermique 26 qui est orientée vers la cavité 12 du casque 10.

La plaque de dissipation thermique 26 est réalisée dans un matériau opaque à la lumière de sorte que la lumière ne peut traverser le casque 10 dans une zone couverte par la couche de dissipation thermique 26. Par exemple, la plaque est une plaque d'aluminium. De préférence, le bandeau 40, la paroi de coque extérieure 22 étant réalisées dans des matériaux translucides, la paroi de coque extérieure 22 présente sur son bord distal 220 un motif en surépaisseur locale qui empêche localement la lumière de passer de façon à produire un motif lumineux.

Dans le mode de réalisation préféré de l'invention, le bandeau 40 est agencé sur le casque 10 de telle sorte que le bandeau 40 ne peut être retiré sans démontage des deux parois de coque 22, 24 entre elles.

De préférence, les deux parois de coque 22, 24 sont assemblées l'une avec l'autre par des moyens 250 de liaison permanents (irréversibles) ou libérales (réversibles).

Dans un mode de réalisation préféré, les moyens de liaison comprennent une pluralité d'éléments de fixation 250, par exemple espacés les uns des autres. Ces éléments de fixation 250 présentent des profils géométriques complémentaires pouvant être assemblés mécaniquement l'un à l'autre, de manière réversible ou irréversible. On entend par assemblage irréversible, un assemblage inviolable par sa résistance physique et mécanique ou par son inertie chimique (en cas de collage par exemple).

Dans le mode de réalisation préféré de l'invention, l'assemblage des deux parois de coque 22, 24 entre elles est irréversible. Il s'agit d'un assemblage par exemple par emboîtement réciproque par clipsage ou par encliquetage agencé de manière à ce qu'il ne soit pas possible de séparer les deux parois 22, 24 sans endommager les moyens de liaison 250 dans des zones de rupture. Ceci empêche un utilisateur de démonter involontairement le casque 10. Ceci permet de garantir que le bandeau 40 ne peut être retiré du casque 10 sans une dégradation irréversible du corps du casque 10.

Par exemple, les moyens de liaisons 250 comprennent des saillies 254 prévues sur l'une 24 des parois lesquelles pénètrent dans des évidements correspondants de l'autre 22 des parois. De préférence, les saillies 254 se présentent sous forme de clips. En outre, les évidements possèdent une denture complémentaire (non illustrée) destinée à coopérer avec les saillies 254 de la paroi 24.

En outre, de préférence, les deux parois 22, 24 sont accouplées entre elles par les clips 254, par exemple en matière plastique et comprenant par exemple des orifices 252 coïncidant des deux parois 22, 24 permettant l'insertion d'un élément de fixation des parois 22, 24 entre elles.

L'insertion d'un élément de fixation (non illustré) permet de contraindre la vitre intérieure 24 formée par la paroi de coque intérieure 24 contre la paroi de coque extérieure 22 par exemple par vissage. Les éléments de fixation sont choisis par exemple parmi : des vis, des boulons, un sertissage, un rivet, un scellage.

On va maintenant décrire les principaux aspects d'un casque de photothérapie selon l'invention en référence aux figures 1 à 5.

Initialement, le casque 10 se présente en plusieurs pièces séparées, une paroi de coque extérieure 22, une paroi de coque intérieure 24, une plaque de dissipation thermique 26, une source de lumière 16, un bandeau d'ajustement 40.

Le bandeau d'ajustement 40 peut être sélectionné par exemple parmi un groupe de trois bandeaux correspondant à trois circonférences de tête différentes correspondant à trois catégories dimensionnelles différentes : une petite dimension, une dimension moyenne et une grande dimension. C'est donc seulement au moment de l'assemblage du bandeau 40 au corps du casque 10 que se définit la catégorie dimensionnelle du casque 10.

L'ordre de montage est le suivant : la plaque de dissipation thermique 26 et la source de lumière 16 sont positionnés à l'intérieur de la concavité de la paroi de coque extérieure 22. Le bandeau 40 est prépositionné en étant relié à la paroi de coque extérieure 22 grâce au talon d'ancrage 48 qui est inséré dans la rainure périphérique 224 et le reste du bandeau 40 vient se positionner naturellement dans sa position de destination finale par élasticité et mémoire de forme.

La vitre formée par la paroi de coque intérieure 24 est alors assemblée sur la paroi de coque extérieure 22 de sorte que lors du vissage de la vitre 24 sur la paroi de coque extérieure 22, le bandeau 40 est contraint par son bourrelet 44 entre les reliefs des deux parois de coque 22, 24 et immobilisé dans sa position finale.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Casque (10) de traitement du cuir chevelu d'un utilisateur par photothérapie, le casque (10) comprenant des parois de coque extérieure (22) et intérieure (24) s'étendant chacune d'une portion de sommet proximale à un bord distal (220, 240) et définissant une fois assemblées, une bordure périphérique (200) du casque (10) qui délimite une ouverture de passage de la tête de l'utilisateur et une source de lumière disposée entre les deux parois (22, 24) émettant vers l'intérieur du casque (10), **caractérisé en ce qu'**il comprend un bandeau circonférentiel (40) d'ajustement du casque (10) à un tour de tête de l'utilisateur, accroché à la bordure périphérique (200), ledit bandeau (40) comprenant un corps pourvu d'une portion de déformation (46) s'étendant radialement vers l'intérieur du casque (10) et étant configurée pour se déformer entre une position de repos déployée radialement vers l'intérieur du casque (10) et une position tassée entre le casque et la tête de l'utilisateur en configuration d'utilisation, le bandeau (40) étant monté sur le casque (10) de telle sorte que le bandeau (40) ne peut être démonté avant démontage des deux parois de coque (22, 24) entre elles..

2. Casque (10) selon la revendication précédente, dans lequel la portion de déformation (46) est creuse.

3. Casque (10) selon l'une quelconque des revendications précédentes, dans lequel la portion de déformation (46) se prolonge vers l'intérieur du casque (10) par un bourrelet (44) de rattachement qui est pincé entre des reliefs des bords distaux (220, 240) des parois de coque (22, 24).

4. Casque (10) selon l'une quelconque des revendications précédentes, dans lequel la paroi intérieure (24) comprend sur sa face arrière une nervure en saillie circonférentielle (242) qui forme un épaulement d'appui (246) au niveau du bord distal (240) de telle sorte que le bourrelet (44) est pris en étau à l'intérieur de cet épaulement (246) par la paroi de coque intérieure (24) contre un relief (228) de la paroi de coque extérieure (22).

5. Casque (10) selon la revendication précédente, dans lequel le bord distal (220) de la paroi de coque extérieure (22) est replié en extrémité en saillie vers l'intérieur pour former un rebord (226) s'étendant radialement vers l'intérieur pour former un épaulement d'appui (228) de telle sorte que le bourrelet (44) est pris en étau à l'intérieur de cet épaulement (228) par la paroi de coque extérieure (22) contre un relief (246) de la paroi de coque intérieure (24).

6. Casque (10) selon l'une quelconque des revendications précédentes, dans lequel la portion de déformation (46) se prolonge vers l'extérieur du casque (10) par un talon d'ancrage extérieur (48) qui est retenu par un relief (224) ménagé sur une face arrière de la paroi de coque extérieure (22).

7. Casque (10) selon l'une quelconque des revendications précédentes, dans lequel le talon d'ancrage (48) est formé par une bordure d'extrémité libre (480) recourbée en U qui vient s'encastrer radialement dans une rainure circonférentielle ménagée sur une face extérieure de la paroi de coque extérieure (22).

8. Casque (10) selon l'une quelconque des revendications précédentes, dans lequel le bandeau (40) d'ajustement est configuré pour être prépositionné en étant retenu de façon libérable sur la paroi de coque extérieure (22) dans une position de destination finale, avant assemblage de la paroi de coque intérieure (22) à la paroi de coque extérieure (24) pour bloquer le bandeau (40) dans ladite position.

9. Casque (10) selon l'une quelconque des revendications précédentes, dans lequel le bandeau (40) est réalisé dans un matériau déformable et résilient de dureté shore A comprise entre 25 et 95 shore.

10. Casque (10) selon l'une quelconque des revendications précédentes, comprenant une plaque (26) d'interface de dissipation thermique réalisée dans un matériau opaque à la lumière qui s'étend entre les deux parois de coque (22, 24).

11. Casque (10) selon l'une quelconque des revendications précédentes, dans lequel le bandeau (40), la paroi de coque extérieure (22) sont réalisées dans des matériaux translucides, la paroi de coque extérieure (22) présente sur son bord distal (220) un motif en surépaisseur locale qui empêche localement la lumière de passer de façon à produire un motif lumineux.

12. Casque (10) selon l'une quelconque des revendications précédentes, dans lequel les deux parois de coque (22, 24) sont assemblées l'une avec l'autre par des moyens (250) de liaison permanents ou libérales.

13. Ensemble comprenant un casque (10) selon l'une quelconque des revendications précédentes et un groupe de bandeaux (40) avec une pluralité de circonférences intérieures différentes définissant autant de catégories dimensionnelles différentes, le bandeau (40) du casque (10) étant choisi dans ledit groupe et assemblé sur le casque (10) pour définir la catégorie dimensionnelle du casque (10).
